# FASCICULE DE BREVET EUROPEEN

(11) **EP 1 629 865 B1**
(45) Date de publication et mention de la délivrance du brevet: **08.10.2008**
(21) Numéro de dépôt: 05291625.1
(22) Date de dépôt: 29.07.2005
(51) Int. Cl.: A61Q 5/12, A61K 8/06, A61K 8/92, A61K 8/81, A61K 8/37, A61K 8/41, A61K 8/45, A61K 8/34, A61K 8/39, A61K 8/60, A61K 8/35

(54) **Emulsion eau-dans-huile, comprenant une huile non-volatile non-siliconée, un tensioactif cationique, une polyoléfine à partie(s) polaire(s), et un alkylmonoglycoside ou alkylpolyglycoside.**
Wasser-in-Öl Emulsion mit einem nicht-flüchtigen Öl ohne Silicongruppen, einem kationischen Tensid, einem Polyolefin mit polaren Anteilen und einem Alkylmonoglycosid oder einem Alkylpolyglycosid.
Water-in-oil emulsion comprising a non-volatile non-siliconized oil, a cationic surfactant, a polyolfin with polar areas, and an alkylmonoglycosid or an alkylpolyglycoside

(30) Priorité: 02.08.2004 FR 0408539
(43) Date de publication de la demande: 01.03.2006
(73) Titulaire: L'ORÉAL, 75008 Paris (FR)
(72) Inventeur: Fack, Geraldine, 92300 Levallois-Perret (FR); Pourille-Grethen, Chrystel, 92110 Clichy (FR)
(74) Mandataire: Casalonga, Axel

(56) Documents cités:
- DE-A1- 4 301 820
- DE-A1- 4 405 510
- DE-A1- 10 147 650
- DE-A1- 19 710 155
- FR-A- 2 828 100
- FR-A- 2 830 773
- FR-A- 2 834 888
- FR-A- 2 841 139
- FR-A- 2 847 831
- CAREY J M ET AL: "IMPROVED HIGH INTERNAL PHASE EMULSIONS USING ALKENYL SUCCINIC ANHYDRIDE BASED EMULSIFIERS" PROCEEDINGS WORLD SURFACTANTS CONGRESS - COMPTES-RENDUS CONGRES MONDIAL DES AGENTS TENSIOACTIFS - KONGRESSBERICHTE WELT TENSID KONGRESS, XX, XX, vol. 2, 29 mai 2000 (2000-05-29), pages 883-888, XP002950021
- KARHE J ET AL: "ALKYLPOLYGLYCOSIDE EIN NEUES KONZEPT FUR PFLEGE UND VERTRAGLICHKEIT IN DER KOSMETIK" SOFW-JOURNAL SEIFEN, OELE, FETTE, WACHSE, VERLAG FUR CHEMISCHE INDUSTRIE, H. ZIOLKOWSKY K.G. AUGSBURG, DE, vol. 121, no. 8, 1 juillet 1995 (1995-07-01), pages 598,600-601,604, XP000514057 ISSN: 0942-7694
- ANONYMOUS: "New cosmetic formulations" RESEARCH DISCLOSURE, KENNETH MASON PUBLICATIONS, HAMPSHIRE, GB, vol. 443, no. 43, mars 2001 (2001-03), XP007127759 ISSN: 0374-4353

## Description

La présente invention est relative à une composition de traitement cosmétique des cheveux, de type émulsion eau-dans-huile, comprenant dans un milieu cosmétiquement acceptable, au moins une huile non-volatile non-siliconée, au moins un tensioactif cationique, au moins une polyoléfine à partie(s) polaire(s), et au moins un alkylmonoglycoside ou alkylpolyglycoside, et à un procédé de traitement cosmétique des cheveux.

Les émulsions eau-dans-huile, notamment exemptes de composés siliconés, sont utilisées couramment en cosmétique et en particulier pour le soin de la peau car elles permettent de former un film lipidique à la surface de la peau la protégeant ainsi des agressions extérieures et prévenant la perte d'eau transépidermique.

Cependant, ces émulsions sont très peu utilisées dans le domaine capillaire, et en particulier celui du conditionnement des cheveux. En effet, elles présentent deux inconvénients majeurs, à savoir de ne pas pouvoir être rincées facilement et totalement, conduisant à un résidu gras inesthétique, et de ne pas permettre l'obtention d'un bon conditionnement du cheveu. En effet, on obtient généralement des cheveux ternes, collés et sales, et on observe un ramollissement de la fibre capillaire. Les cheveux sont en outre difficiles à démêler.

La demanderesse a découvert de manière surprenante que l'introduction d'une association particulière de tensioactifs et de polyoléfine à partie(s) polaire(s) dans une telle émulsion eau-dans-huile permettait non seulement d'améliorer sa rinçabilité et d'obtenir de bonnes propriétés cosmétiques, mais aussi d'obtenir une émulsion eau-dans-huile stable. Cette association particulière est constituée par au moins un tensioactif cationique, au moins une polyoléfine à partie(s) polaire(s), et au moins un (alkyl en C₁₂₋₃₀)monoglycoside ou (alkyl en C₁₂₋₃₀)polyglycoside.

La présente invention a donc pour objet une composition de traitement des cheveux, de type émulsion eau-dans-huile, comprenant dans un milieu cosmétiquement acceptable, au moins une huile non-volatile non-siliconée, au moins un tensioactif cationique, au moins une polyoléfine à partie(s) polaire(s), et au moins un (alkyl en C₁₂₋₃₀)monoglycoside ou (alkyl en C₁₂₋₃₀)polyglycoside.

Un autre objet de l'invention consiste en un procédé de traitement cosmétique des cheveux mettant en oeuvre une composition selon l'invention telle que décrite ci-dessous.

L'invention a encore pour objet une utilisation de la composition selon l'invention pour le conditionnement des cheveux et notamment comme après-shampoing.

D'autres objets, caractéristiques, aspects et avantages de l'invention apparaîtront encore plus clairement à la lecture de la description et de l'exemple qui suit.

Selon l'invention, la composition de traitement cosmétique des cheveux, de type émulsion eau-dans-huile, comprend dans un milieu cosmétiquement acceptable, au moins une huile non-volatile non-siliconée, au moins un tensioactif cationique, au moins une polyoléfine à partie(s) polaire(s), et au moins un alkylmonoglycoside ou alkylpolyglycoside.

Par "milieu cosmétiquement acceptable", on entend un milieu compatible avec les cheveux.

Par "huile", on entend tout milieu non aqueux liquide à température ambiante (25°C±3°C) et sous pression atmosphérique, ayant une solubilité dans l'eau à 25°C inférieure à 0,5%.

Par "huile non-volatile", on entend une huile ayant une pression de vapeur à température ambiante (25°C±3°C) inférieure à 2,66 Pa (0,02 mm de mercure).

Les huiles non-volatiles non-siliconées utilisables dans la présente invention sont notamment choisies parmi les huiles végétales, les huiles animales, les huiles minérales, les huiles synthétiques, les esters d'acide gras, et leurs mélanges.

Comme huile végétale, on peut notamment mentionner l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot et l'huile de calophyllum.

Comme huile animale, on peut notamment citer le perhydrosqualène.

La composition selon l'invention peut également comprendre une ou plusieurs huiles minérales telles qu'une huile de paraffine et l'huile de vaseline.

La composition selon l'invention peut également comprendre une ou plusieurs huiles synthétiques telles que le squalane, les poly(α-oléfines) comme l'isododécane ou l'isohexadécane, les huiles végétales transestérifiées et les huiles fluorées.

La composition selon l'invention peut également comprendre un ou plusieurs esters gras, tels que par exemple, les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le mombre total d'atomes de carbone de l'ester étant supérieur à 10. A titre d'exemples, on peut notamment citer l'huile de Purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

Les huiles particulièrement préférées dans la composition selon l'invention sont notamment choisies parmi l'huile d'avocat, l'isododécane, le myristate d'isopropyle et la cire liquide de jojoba.

L'huile ou les huiles telles que décrites ci-dessus sont notamment présentes dans la composition selon l'invention en une quantité allant de 0,1 à 30 % en poids, de préférence de 1 à 20 % en poids, et mieux encore de 5 à 15% en poids par rapport au poids total de la composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux répondant à la formule générale (I) suivante : 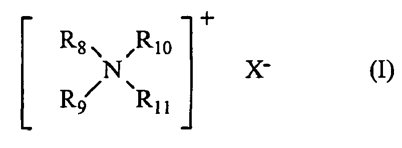 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C₁₋₃₀, alcoxy en C₁₋₃₀, polyoxyalkylène (C₂-C₆), alkylamide en C₁₋₃₀, alkyl(C₁₂-C₂₂)amidoalkyle(C₂-C₆), alkyl(C₁₂₋C₂₂)acétate, et hydroxyalkyle en C₁₋₃₀ ; X est un anion choisi dans le groupe des halogénures, phosphates, acétates, lactates, alkyl(C₂₋C₆)sulfates, alkyl- ou alkylaryl-sulfonates
   Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.
- les sels d'ammonium quaternaire de l'imidazoline, comme par exemple ceux de formule (II) suivante : 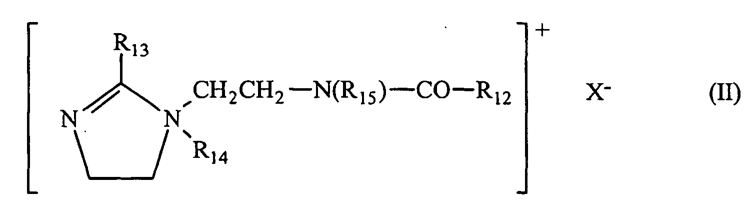 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (III) : 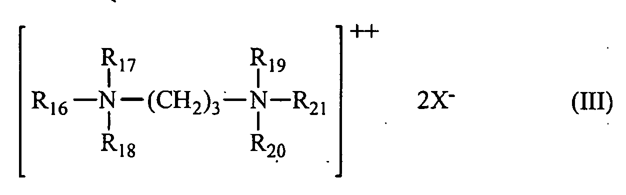 dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 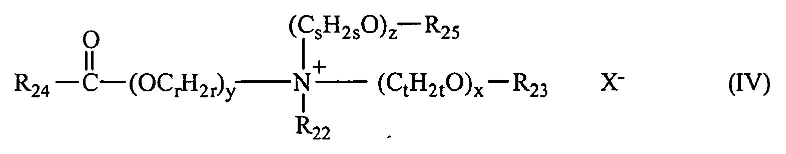 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 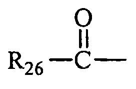
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 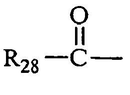
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 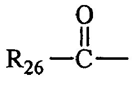
   - les radicaux méthyle, éthyle ou hydrocarbonés en C₁₄-C₂₂,
   - l'atome d'hydrogène ;
- R₂₅ est choisi parmi :
   - le radical 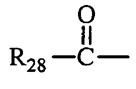
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques
ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Les tensioactifs cationiques particulièrement préférés dans la composition de l'invention sont choisis parmi les sels d'ammonium quaternaire, et en particulier parmi le chlorure de béhényltriméthylammonium et le chlorure de cétyltriméthylammonium.

La composition de traitement cosmétique des cheveux comprend de préférence le ou les tensioactifs cationiques en une quantité allant de 0,1 à 20 % en poids, mieux encore de 0,2 à 10 % en poids, et encore plus préférentiellement de 0,5 à 8 % en poids par rapport au poids total de la composition.

Les polyoléfines à partie(s) polaire(s) utilisables dans l'invention sont connues dans d'autres domaines. Ainsi, elles sont décrites par exemple dans les documents US-A-5,129,972 et US-A-4,919,179, comme stabilisants d'émulsions explosives.

Par ailleurs, ces composés sont connus comme stabilisants de compositions fertilisantes (voir les documents US-A-5,518,517 et US-A-5,858,055) en vue d'obtenir un relargage contrôlé des substances fertilisantes.

Les polyoléfines à partie(s) polaire(s) utilisées dans la composition de l'invention sont constituées d'une partie apolaire polyoléfinique et d'au moins une partie polaire. Elles peuvent présenter une structure de type bloc ou peigne.

La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène. Ces polyoléfines sont hydrogénées ou non.

Par ailleurs, les polyoléfines à partie(s) polaire(s) utilisées dans la composition de l'invention comportent au moins une partie polaire. Cette partie polaire leur confère des propriétés amphiphiles. Ainsi, ces polyoléfines à partie(s) polaire(s) abaissent la tension interfaciale (eau/huile) d'au moins 10 mN/m quand ils sont présents à une concentration de 0,01% en poids par rapport au poids total de la phase huileuse. Par exemple, la polyoléfine à terminaison succinique commercialisée sous la dénomination Lubrizol 2724 par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1% de MgSO₄, et d'une phase huileuse comportant un mélange d'huiles (isohexadécane/polyisobutène hydrogéné/silicone volatile dans un rapport 8/6/4).

La partie polaire des polyoléfines à partie(s) polaire(s) de l'invention peut être anionique, cationique, non ionique, zwitterionique ou amphotère. Elle est par exemple constituée de polyalkylène-glycols ou de polyalkylène-imines, ou encore d'acides ou de diacides carboxyliques, de leurs anhydrides ou de leurs dérivés tels que leurs esters, leurs amides et leurs sels, et leurs mélanges. Les polyoléfines à partie polaire acide carboxylique peuvent être par exemple issues de la réaction entre une polyoléfine et au moins un acide ou anhydride carboxylique éventuellement totalement ou partiellement salifié, choisi dans le groupe comprenant l'acide maléique, l'anhydride maléique, l'acide fumarique, l'acide itaconique, l'acide citraconique, l'acide mésaconique, l'acide aconitique, l'acide
ou l'anhydride succinique, leurs dérivés esters ou amides, et leurs mélanges.

De préférence, la partie polaire est constituée par l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique, ou bien encore par un polyoxyéthylène.

Les polyoléfines à partie(s) polaire(s) polyoxyéthylène peuvent être par exemple choisies parmi les polymères diblocs polyisoprènepolyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p.1582-1586).

Les polyoléfines à partie polaire acide ou anhydride succinique peuvent être choisies notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799 et US-A-4,919,179. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être éventuellement modifiée, c'est-à-dire estérifiée, amidifiée ou sous forme de sel. Elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de monoamines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatiques, cycloaliphatiques, aromatiques et hétérocycliques. Les polyoléfines à terminaison succinique modifiée (estérifiée ou amidifiée) et leur procédé de préparation sont décrits en particulier dans le document US-A-4,708,753.

Comme polyoléfines à terminaison succinique, on peut citer notamment les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol.

Un autre exemple de polyoléfine à partie(s) polaire(s) utilisable dans l'invention est le produit de la réaction de l'anhydride maléique avec le polyisobutylène, tel que le produit commercialisé sous la dénomination Glissopal SA par la société BASF.

La polyoléfine à partie(s) polaire(s) particulièrement préférée est un produit réactionnel de l'anhydride polyisobuténylsuccinique avec de la diéthyléthanolamine. Ce produit est vendu par exemple sous la dénomination Lubrizol 5603 par la société Lubrizol, et peut être représenté par la formule suivante : 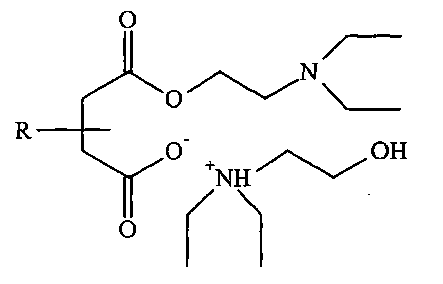 dans laquelle R représente un groupe polyisobutényle de masse moléculaire en poids de 1000.

Le ou les polyoléfine à partie(s) polaire(s), telles que définies ci-dessus sont contenues de préférence en une quantité allant de 0,01 à 10 % en poids, de préférence de 0,1 à 5 % en poids, et mieux encore de 0,2 à 3 % en poids par rapport au poids total de la composition.

Les alkylmonoglycosides ou alkylpolyglycosides particulièrement préférés dans l'invention sont ceux dont le groupe alkyle comporte de 16 à 24 atomes de carbone.

A titre d'exemples particulièrement préférés, on peut notamment citer l'arachidylglycoside

Le ou les (alkyl en C₁₂₋₃₀)monoglycosides ou (alkyl en C₁₂₋₃₀)polyglycosides sont contenus de préférence en une quantité allant de 0,01 à 10 % en poids, de préférence de 0,02 à 5 % en poids, et mieux encore de 0,05 à 1 % en poids par rapport au poids total de la composition.

De préférence, le rapport pondéral huile(s)/polyoléfine(s) à partie(s) polaire(s) des compositions de l'invention est compris entre 3 et 100, mieux encore entre 10 et 75 et encore plus préférentiellement entre 15 et 40.

Le milieu aqueux cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

Les compositions selon l'invention peuvent comprendre en outre au moins un alcool gras en C₁₄₋₃₀, et de préférence au moins l'un des alcools myristylique, cétylique, stéarylique, arachidylique, béhénylique et érucylique. Ils sont généralement présents en une quantité inférieure à 10 % en poids, de préférence allant de 0,01 à 5 % en poids, et mieux encore de 0,05 à 1,5 % en poids par rapport au poids total de la composition.

Les compositions selon l'invention peuvent également comprendre en outre au moins une huile siliconée bien connue dans la technique, en une quantité inférieure à 10 % en poids, de préférence allant de 0,01 à 8%, et encore plus préférentiellement de 0,1 à 5% en poids par rapport au poids total de la composition.

A titre d'exemples d'huile siliconée, on peut notamment citer les polydiméthylsiloxanes linéaires ou cycliques.

Les compositions selon l'invention peuvent également contenir au moins un additif tel qu'un polymère cationique, anionique, non ionique ou amphotère ; un épaississant polymérique naturel ou synthétique, anionique, amphotère, zwittérionique, non ionique ou cationique, associatif ou non ; un épaississant non polymérique comme un électrolyte ou un sucre ; un nacrant ; un opacifiant ; un filtre solaire ; un parfum ; un colorant ; une particule organique ou minérale ; un conservateur ; ou un agent de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 50 % en poids par rapport au poids total de la composition.

Les compositions selon l'invention peuvent se présenter sous forme de liquides fluides ou épaissis, de gels, de crèmes, ou d'émulsions simples ou multiples.

Les compositions peuvent être utilisées, par exemple, dans des shampoings, produits de coloration ou de décoloration ou de permanente, produits de coiffage, soins rincés, masques de soin profond, gels douches, lotions ou crèmes de traitement du cuir chevelu, ou encore déposées sur des lingettes.

La présente invention concerne également un procédé de traitement cosmétique des cheveux qui consiste à appliquer une quantité efficace d'une composition telle que décrite ci-dessus, sur les cheveux, à effectuer un éventuel rinçage après un éventuel temps de pose.

Selon un mode de réalisation préféré de l'invention, la composition peut être utilisée pour le conditionnement des cheveux et plus particulièrement comme après-shampoing.

L'exemple suivant est donné à titre illustratif de la présente invention.

### EXEMPLE

On a préparé l'émulsion eau-dans-huile en mélangeant les ingrédients indiqués dans le tableau ci-dessous dans les proportions indiquées en % en poids par rapport au poids total de l'émulsion.

| | Ex. 1 |
|---|---|
| Isoparaffine en C₁₁₋₁₂ | 3,55 |
| Huile d'avocat | 1,2 |
| Polyoléfine à partie polaire⁽¹⁾ | 0,25 |
| Arachidylglucoside (à 15 % de MA)⁽²⁾ | 0,2 MA |
| Chlorure de béhényltriméthylammonium (à 80 % de MA) ⁽³⁾ | 2 MA |
| Eau qsp | 100 |

| | |
|---|---|
| MA : Matière Active ⁽¹⁾ : vendu sous le nom commercial Lubrizol TM 5603 par LUBRIZOL ⁽²⁾ : vendu sous le nom commercial Montanov 202 par SEPPIC ⁽³⁾ : vendu sous le nom commercial Genamin KDMP par CLARIANT | |

On a appliqué l'émulsion sur des cheveux sensibilisés.

On observe alors une bonne rinçabilité et un bon conditionnement de la fibre.

## Revendications

1. Composition de traitement cosmétique des cheveux, de type émulsion eau-dans-huile, **caractérisée en ce qu'**elle comprend dans un milieu cosmétiquement acceptable, au moins une huile non-volatile non-siliconée, au moins un tensioactif cationique, au moins une polyoléfine à partie(s) polaire(s), et au moins un (alkyl en C₁₂₋₃₀) monoglycoside ou (alkyl en C₁₂₋₃₀)polyglycoside.

2. Composition de traitement cosmétique des cheveux selon la revendication 1, **caractérisée en ce que** l'huile non-volatile non-siliconée est une huile végétale, une huile animale, une huile minérale, une huile synthétique, un ester d'acide gras ou un de leurs mélanges.

3. Composition de traitement cosmétique des cheveux selon la revendication 2, **caractérisée en ce que** l'huile végétale est choisie parmi l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot et l'huile de calophyllum.

4. Composition de traitement cosmétique des cheveux selon la revendication 2, **caractérisée en ce que** l'huile animale est le perhydrosqualène.

5. Composition de traitement cosmétique des cheveux selon la revendication 2, **caractérisée en ce que** l'huile minérale est choisie parmi l'huile de paraffine et l'huile de vaseline.

6. Composition de traitement cosmétique des cheveux selon la revendication 2, **caractérisée en ce que** l'huile synthétique est choisie parmi le squalane, les poly (α-oléfines), les huiles végétales transestérifiées et les huiles fluorées.

7. Composition de traitement cosmétique des cheveux selon la revendication 2, **caractérisée en ce que** l'ester gras est choisi parmi l'huile de Purcellin, le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

8. Composition de traitement cosmétique des cheveux selon la revendication 1 ou 2, **caractérisée en ce que** l'huile non-volatile non-siliconée est choisie parmi l'huile d'avocat, l'isododécane, le myristate d'isopropyle et la cire liquide de jojoba.

9. Composition de traitement cosmétique des cheveux selon l'une quelconque des revendications précédentes, **caractérisée en ce qu'**elle comprend l'huile ou les huiles en une quantité allant de 0,1 à 30 % en poids, de préférence de 1 à 20% en poids, et mieux encore de 5 à 15% en poids par rapport au poids total de la composition.

10. Composition de traitement cosmétique des cheveux selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

11. Composition de traitement cosmétique des cheveux selon la revendication 10, **caractérisée en ce que** les sels d'ammonium quaternaire sont choisis parmi :
- ceux répondant à la formule générale (I) suivante : 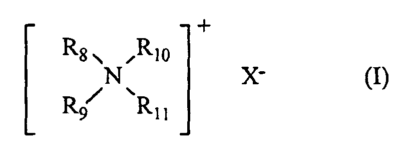
dans laquelle les radicaux R₈ à R₁₁, qui peuvent être identiques ou différents, représentent un radical aliphatique, linéaire ou ramifié, comportant de 1 à 30 atomes de carbone, ou un radical aromatique ; X est un anion choisi dans le groupe des halogénures, phosphates, acétates, lactates, alkyl (C₂-C₆) sulfates, alkyl- ou alkylaryl-sulfonates ;
- les sels d'ammonium quaternaire de l'imidazoline ;
- les sels de diammonium quaternaire de formule (III) : 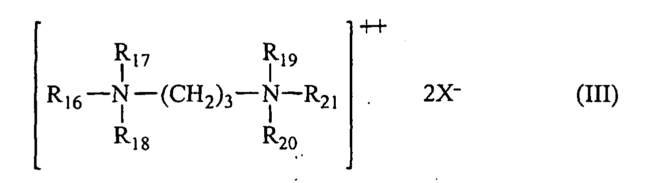
dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates ;
- les sels d'ammonium quaternaire contenant au moins une fonction ester.

12. Composition de traitement cosmétique des cheveux selon la revendication 10 ou 11, **caractérisée en ce que** le tensioactif cationique est choisi parmi les chlorures de béhényltriméthylammonium et de cétyltriméthylammonium.

13. Composition de traitement cosmétique des cheveux selon l'une quelconque des revendications précédentes, **caractérisée en ce qu'**elle comprend le(s) tensioactif(s) cationique(s) en une quantité allant de 0,1 à 20 % en poids, de préférence de 0,2 à 10 % et encore plus préférentiellement de 0,5 à 8% en poids par rapport au poids total de la composition.

14. Composition de traitement cosmétique des cheveux selon l'une quelconque des revendications précédentes, **caractérisée en ce que** la polyoléfine à partie(s) polaire(s) comporte une partie apolaire polyoléfinique comprenant au moins 40 atomes de carbone, et de préférence de 60 à 700 atomes de carbone.

15. Composition de traitement cosmétique des cheveux selon la revendication 14, **caractérisée en ce que** la partie apolaire polyoléfinique est choisie parmi les oligomères, les polymères et/ou les copolymères d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène.

16. Composition de traitement cosmétique des cheveux selon l'une quelconque des revendications précédentes, **caractérisée en ce que** la partie polaire est anionique, cationique, non ionique, zwitterionique ou amphotère.

17. Composition de traitement cosmétique des cheveux selon l'une quelconque des revendications précédentes, **caractérisée en ce que** la partie polaire est constituée de polyalkylène-glycols, de polyalkylène-imines, d'acides ou de diacides carboxyliques, de leurs anhydrides ou dérivés tels que esters, amides et sels, et leurs mélanges.

18. Composition de traitement cosmétique des cheveux selon la revendication 17, **caractérisée en ce que** la partie polaire est choisie dans le groupe comprenant le polyoxyéthylène, l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique.

19. Composition de traitement cosmétique des cheveux selon l'une quelconque des revendications précédentes, **caractérisée en ce que** la polyoléfine à partie(s) polaire(s) est un polyisobutylène à terminaison succinique éventuellement modifiée.

20. Composition de traitement cosmétique des cheveux selon la revendication 17, **caractérisée en ce que** la polyoléfine à partie(s) polaire(s) est le produit de la réaction de l'anhydride maléique avec le polyisobutylène.

21. Composition de traitement cosmétique des cheveux selon l'une quelconque des revendications précédentes, **caractérisée en ce qu'**elle comprend la ou le(s) polyoléfine(s) à partie(s) polaire(s) en une quantité allant de 0,01 à 10 % en poids, de préférence de 0,1 à 5 % en poids par rapport au poids total de la composition.

22. Composition de traitement cosmétique des cheveux selon l'une quelconque des revendications précédentes, **caractérisée en ce que** l'(alkyl en C₁₂₋₃₀) monoglycoside ou (alkyl en C₁₂₋₃₀) polyglycoside est choisi parmi ceux dont le groupe alkyle comporte de 16 à 24 atomes de carbone.

23. Composition de traitement cosmétique des cheveux selon l'une quelconque des revendications précédentes, **caractérisée en ce qu'**elle comprend le(s) (alkyl en C₁₂₋₃₀) monoglycoside(s) ou (alkyl en C₁₂-₃₀) polyglycoside(s) en une quantité allant de 0,01 à 10 % en poids, de préférence de 0,02 à 5 % en poids par rapport au poids total de la composition.

24. Composition de traitement cosmétique des cheveux selon l'une quelconque des revendications précédentes, **caractérisée en ce que** le rapport pondéral huile(s)/polyoléfine(s) à partie(s) polaire(s) est compris entre 3 et 100, de préférence entre 10 et 75.

25. Composition de traitement cosmétique des cheveux selon l'une quelconque des revendications précédentes, **caractérisée en ce qu'**elle comprend en outre au moins un alcool gras en C₁₄₋₃₀.

26. Composition de traitement cosmétique des cheveux selon la revendication 25, **caractérisée en ce que** l'alcool gras en C₁₄₋₃₀ est choisi parmi les alcools myristylique, cétylique, stéarylique, arachidylique, béhénylique et érucylique.

27. Composition de traitement cosmétique des cheveux selon la revendication 25 ou 26, **caractérisée en ce qu'**elle comprend le(s) alcool(s) gras en une quantité inférieure à 10 % en poids par rapport au poids total de la composition.

28. Composition de traitement cosmétique des cheveux selon l'une quelconque des revendications précédentes, **caractérisée en ce que** le milieu cosmétiquement acceptable comprend l'eau ou un mélange d'eau et d'au moins un solvant cosmétiquement acceptable.

29. Composition de traitement cosmétiqué des cheveux selon la revendication 28, **caractérisée en ce que** le solvant cosmétiquement acceptable est choisi parmi les alcools inférieurs en C₁-C₄, les polyols, les éthers de polyols, les alcanes en C₅-C₁₀, les cétones en C₃₋₄, les acétates d'alkyle en C₁-C₄, le diméthoxyéthane et le diéthoxyéthane.

30. Composition de traitement cosmétique des cheveux selon l'une quelconque des revendications précédentes, **caractérisée en ce qu'**elle comprend en outre au moins une huile siliconée en une quantité inférieure à 10 % en poids par rapport au poids total de la composition.

31. Composition de traitement cosmétique des cheveux selon l'une quelconque des revendications précédentes, **caractérisée en ce qu'**elle comprend au moins un additif choisi parmi les polymères cationiques, anioniques, non ioniques ou amphotères ; les épaississants polymériques naturels ou synthétiques, anioniques, amphotères, zwittérioniques, non ioniques ou cationiques, associatifs ou non ; les épaississants non polymériques ; les nacrants ; les opacifiants ; les filtres solaires ; les parfums ; les colorants ; les particules organiques ou minérales ; les conservateurs ; et les agents de stabilisation du pH.

32. Procédé de traitement cosmétique des cheveux, **caractérisé en ce que** l'on applique sur les cheveux une composition de traitement cosmétique selon l'une quelconque des revendications précédentes.

33. Utilisation d'une composition de traitement cosmétique selon l'une quelconque des revendications 1 à 31, pour le conditionnement des cheveux.

34. Utilisation d'une composition de traitement cosmétique selon la revendication 33, comme après-shampoing.

## Claims

1. Cosmetic hair treatment composition, of water-in-oil emulsion type, **characterized in that** it comprises, in a cosmetically acceptable medium, at least one non-volatile non-silicone oil, at least one cationic surfactant, at least one polyolefin with polar portion(s), and at least one (C₁₂₋₃₀ alkyl) monoglycoside or (C₁₂₋₃₀ alkyl) polyglycoside.

2. Cosmetic hair treatment composition according to Claim 1, **characterized in that** the non-volatile non-silicone oil is a plant oil, an animal oil, a mineral oil, a synthetic oil or a fatty acid ester, or a mixture thereof.

3. Cosmetic hair treatment composition according to Claim 2, **characterized in that** the plant oil is chosen from sweet almond oil, avocado oil, castor oil, olive oil, liquid jojoba wax, sunflower oil, wheatgerm oil, sesame seed oil, groundnut oil, grapeseed oil, soybean oil, rapeseed oil, safflower oil, coconut oil, corn oil, hazelnut oil, palm oil, apricot kernel oil and beauty-leaf oil.

4. Cosmetic hair treatment composition according to Claim 2, **characterized in that** the animal oil is perhydrosqualene.

5. Cosmetic hair treatment composition according to Claim 2, **characterized in that** the mineral oil is chosen from liquid paraffin and liquid petroleum jelly.

6. Cosmetic hair treatment composition according to Claim 2, **characterized in that** the synthetic oil is chosen from squalane, poly(α-olefins), transesterified plant oils and fluoro oils.

7. Cosmetic hair treatment composition according to Claim 2, **characterized in that** the fatty ester is chosen from purcellin oil, isopropyl myristate, isopropyl palmitate, butyl stearate, hexyl laurate, isononyl isononanoate, 2-ethylhexyl palmitate, 2-hexyldecyl laurate, 2-octyldecyl palmitate, 2-octyldodecyl myristate, isostearyl neopentanoate and tridecyl neopentanoate.

8. Cosmetic hair treatment composition according to Claim 1 or 2, **characterized in that** the non-volatile non-silicone oil is chosen from avocado oil, isododecane, isopropyl myristate and liquid jojoba wax.

9. Cosmetic hair treatment composition according to any one of the preceding claims, **characterized in that** it comprises the oil(s) in an amount ranging from 0.1% to 30% by weight, preferably from 1% to 20% by weight and better still from 5% to 15% by weight relative to the total weight of the composition.

10. Cosmetic hair treatment composition according to any one of the preceding claims, **characterized in that** the cationic surfactants are chosen from optionally polyoxyalkylenated primary, secondary or tertiary fatty amine salts and quaternary ammonium salts, and mixtures thereof.

11. Cosmetic hair treatment composition according to Claim 10, **characterized in that** the quaternary ammonium salts are chosen from:
those corresponding to the general formula (I) below: 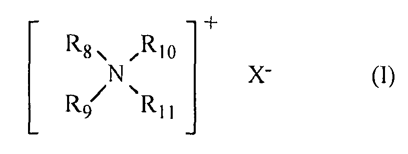
in which the radicals R₈ to R₁₁, which may be identical or different, represent a linear or branched aliphatic radical containing from 1 to 30 carbon atoms, or an aromatic radical; X⁻ is an anion chosen from the group of halides, phosphates, acetates, lactates, (C₂-C₆)alkyl sulfates and alkyl- or alkylaryl-sulfonates;
- quaternary ammonium salts of imidazoline;
- the diquaternary ammonium salts of formula (III): 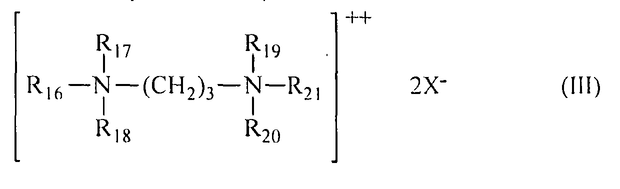
in which R₁₆ denotes an aliphatic radical containing from about 16 to 30 carbon atoms, R₁₇, R₁₈, R₁₉, R₂₀ and R₂₁, which may be identical or different, are chosen from a hydrogen atom and an alkyl radical containing from 1 to 4 carbon atoms, and X⁻ is an anion chosen from the group of halides, acetates, phosphates, nitrates and methyl sulfates;
- quaternary ammonium salts containing at least one ester function.

12. Cosmetic hair treatment composition according to Claim 10 or 11, **characterized in that** the cationic surfactant is chosen from behenyltrimethylammonium chloride and cetyltrimethylammonium chloride.

13. Cosmetic hair treatment composition according to any one of the preceding claims, **characterized in that** it comprises the cationic surfactant(s) in an amount ranging from 0.1% to 20% by weight, preferably from 0.2% to 10% and even more preferably from 0.5% to 8% by weight relative to the total weight of the composition.

14. Cosmetic hair treatment composition according to any one of the preceding claims, **characterized in that** the polyolefin with polar portion(s) comprises a polyolefinic apolar portion containing at least 40 carbon atoms and preferably from 60 to 700 carbon atoms.

15. Cosmetic hair treatment composition according to Claim 14, **characterized in that** the polyolefinic apolar portion is chosen from oligomers, polymers and/or copolymers of ethylene, propylene, 1-butene, isobutene, 1-pentene, 2-methyl-1-butene, 3-methyl-1-butene, 1-hexene, 1-heptene, 1-octene, 1-decene, 1-undecene, 1-dodecene, 1-tridecene, 1-tetradecene, 1-pentadecene, 1-hexadecene, 1-heptadecene and 1-octadecene.

16. Cosmetic hair treatment composition according to any one of the preceding claims, **characterized in that** the polar portion is anionic, cationic, nonionic, zwitterionic or amphoteric.

17. Cosmetic hair treatment composition according to any one of the preceding claims, **characterized in that** the polar portion consists of polyalkylene glycols, polyalkyleneimines, carboxylic or dicarboxylic acids, anhydrides or derivatives thereof such as esters, amides and salts, and mixtures thereof.

18. Cosmetic hair treatment composition according to Claim 17, **characterized in that** the polar portion is chosen from the group comprising polyoxyethylene, succinic acid or anhydride, succinic acid or anhydride esters or amides, alkali metal salts, alkaline-earth metal salts or organic salts of succinic acid or anhydride, or partial salts of succinic acid or anhydride monoesters or monoamides.

19. Cosmetic hair treatment composition according to any one of the preceding claims, **characterized in that** the polyolefin with polar portion(s) is a polyisobutylene with optionally modified succinic end groups.

20. Cosmetic hair treatment composition according to Claim 17, **characterized in that** the polyolefin with polar portion(s) is the product of reaction of maleic anhydride with polyisobutylene.

21. Cosmetic hair treatment composition according to any one of the preceding claims, **characterized in that** it comprises the polyolefin(s) with polar portion(s) in an amount ranging from 0.01% to 10% by weight and preferably from 0.1% to 5% by weight relative to the total weight of the composition.

22. Cosmetic hair treatment composition according to any one of the preceding claims, **characterized in that** the (C₁₂₋₃₀ alkyl)monoglycoside or (C₁₂₋₃₀ alkyl)polyglycoside is chosen from those in which the alkyl group contains from 16 to 24 carbon atoms.

23. Cosmetic hair treatment composition according to any one of the preceding claims, **characterized in that** it comprises the (C₁₂₋₃₀ alkyl)monoglycoside(s) or (C₁₂₋₃₀ alkyl)polyglycoside(s) in an amount ranging from 0.01% to 10% by weight and preferably from 0.02% to 5% by weight relative to the total weight of the composition.

24. Cosmetic hair treatment composition according to any one of the preceding claims, **characterized in that** the weight ratio of oil(s)/polyolefin(s) with polar portion(s) is between 3 and 100 and preferably between 10 and 75.

25. Cosmetic hair treatment composition according to any one of the preceding claims, **characterized in that** it also comprises at least one C₁₄₋₃₀ fatty alcohol.

26. Cosmetic hair treatment composition according to Claim 25, **characterized in that** the C₁₄₋₃₀ fatty alcohol is chosen from myristyl alcohol, cetyl alcohol, stearyl alcohol, arachidyl alcohol, behenyl alcohol and erucyl alcohol.

27. Cosmetic hair treatment composition according to Claim 25 or 26, **characterized in that** it comprises the fatty alcohol(s) in an amount of less than 10% by weight relative to the total weight of the composition.

28. Cosmetic hair treatment composition according to any one of the preceding claims, **characterized in that** the cosmetically acceptable medium comprises water or a mixture of water and of at least one cosmetically acceptable solvent.

29. Cosmetic hair treatment composition according to Claim 28, **characterized in that** the cosmetically acceptable solvent is chosen from C₁-C₄ lower alcohols, polyols, polyol ethers, C₅ -C₁₀ alkanes, C₃₋₄ ketones, C₁-C₄ alkyl acetates, dimethoxyethane and diethoxyethane.

30. Cosmetic hair treatment composition according to any one of the preceding claims, **characterized in that** it also comprises at least one silicone oil in an amount of less than 10% by weight relative to the total weight of the composition.

31. Cosmetic hair treatment composition according to any one of the preceding claims, **characterized in that** it comprises at least one additive chosen from cationic, anionic, nonionic or amphoteric polymers; natural or synthetic, anionic, amphoteric, zwitterionic, nonionic or cationic, associative or non-associative polymeric thickeners; non-polymeric thickeners; nacreous agents; opacifiers; sunscreens; fragrances; dyes; organic or mineral particles; preserving agents; and pH stabilizers.

32. Cosmetic hair treatment process, **characterized in that** a cosmetic treatment composition according to any one of the preceding claims is applied to the hair.

33. Use of a cosmetic treatment composition according to any one of Claims 1 to 31 for conditioning the hair.

34. Use of a cosmetic treatment composition according to Claim 33 as a hair conditioner.

## Patentansprüche

1. Zusammensetzung zur kosmetischen Behandlung der Haare vom Typ einer Wasser-in-Öl-Emulsion, **dadurch gekennzeichnet, dass** sie in einem kosmetisch akzeptablen Medium enthält:
mindestens ein nichtflüchtiges Öl ohne Silicongruppen, mindestens ein kationisches Tensid,
mindestens ein Polyolefin mit einem oder mehreren polaren Teilen, und
mindestens ein C₁₂₋₃₀-Alkylmonoglycosid oder C₁₂₋₃₀-Alkylpolyglycosid.

2. Zusammensetzung zur kosmetischen Behandlung der Haare nach Anspruch 1, **dadurch gekennzeichnet, dass** das nichtflüchtige Öl ohne Silicongruppen ein Pflanzenöl, ein tierisches Öl, ein Mineralöl, ein synthetisches Öl oder ein Fettsäureester oder ein Gemisch dieser Verbindungen ist.

3. Zusammensetzung zur kosmetischen Behandlung der Haare nach Anspruch 2, **dadurch gekennzeichnet, dass** das Pflanzenöl ausgewählt ist unter Süßmandelöl, Avocadoöl, Rizinusöl, Olivenöl, flüssigem Jojobawachs, Sonnenblumenöl, Weizenkeimöl, Sesamöl, Erdnussöl, Traubenkernöl, Sojaöl, Rapsöl, Distelöl, Coprahöl, Maisöl, Haselnussöl, Palmöl, Aprikosenkernöl und Calophyllumöl.

4. Zusammensetzung zur kosmetischen Behandlung der Haare nach Anspruch 2, **dadurch gekennzeichnet, dass** das tierische Öl Perhydrosqualen ist.

5. Zusammensetzung zur kosmetischen Behandlung der Haare nach Anspruch 2, **dadurch gekennzeichnet, dass** das Mineralöl unter Paraffinöl und Vaselinöl ausgewählt ist.

6. Zusammensetzung zur kosmetischen Behandlung der Haare nach Anspruch 2, **dadurch gekennzeichnet, dass** das synthetische Öl unter Squalan, Poly(α-olefinen), umgeesterten Pflanzenölen und fluorierten Ölen ausgewählt ist.

7. Zusammensetzung zur kosmetischen Behandlung der Haare nach Anspruch 2, **dadurch gekennzeichnet, dass** der Fettsäureester ausgewählt ist unter Purcellinöl, Isopropylmyristat, Isopropylpalmitat, Butylstearat, Hexyllaurat, Isononylisononanoat, 2-Ethylhexylpalmitat, 2-Hexyldecyllaurat, 2-Octyldecylpalmitat, 2-Octyldodecylmyristat, Isostearylneopentanoat oder Tridecylneopentanoat.

8. Zusammensetzung zur kosmetischen Behandlung der Haare nach Anspruch 1 oder 2, **dadurch gekennzeichnet, dass** das nichtflüchtige Öl ohne Silicongruppen ausgewählt ist unter Avocadoöl, Isododecan, Isopropylmyristat und flüssigem Jojobawachs.

9. Zusammensetzung zur kosmetischen Behandlung der Haare nach einem der vorhergehenden Ansprüche, **dadurch gekennzeichnet, dass** sie das Öl oder die Öle in einem Mengenanteil von 0,1 bis 30 Gew.-%, bevorzugt von 1 bis 20 Gew.-% und noch günstiger von 5 bis 15 Gew.-% enthält, bezogen auf das Gesamtgewicht der Zusammensetzung.

10. Zusammensetzung zur kosmetischen Behandlung der Haare nach einem der vorhergehenden Ansprüche, **dadurch gekennzeichnet, dass** die kationischen Tenside unter Salzen von primären, sekundären oder tertiären und gegebenenfalls polyoxyalkylenierten Fettaminen und quaternären Ammoniumsalzen sowie Gemischen dieser Verbindungen ausgewählt sind.

11. Zusammensetzung zur kosmetischen Behandlung der Haare nach Anspruch 10, **dadurch gekennzeichnet, dass** die quaternären Ammoniumsalze ausgewählt sind unter
- quaternären Ammoniumsalzen der nachstehenden allgemeinen Formel (I): 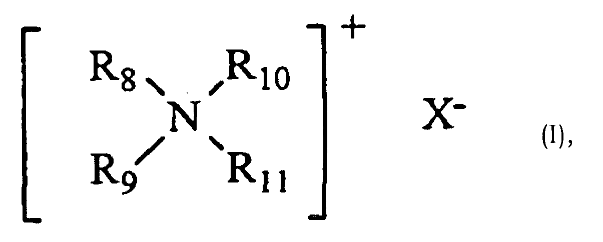 in der bedeuten:
R₈ bis R₁₁, die gleich oder verschieden sein können, eine geradkettige oder verzweigte aliphatische Gruppe mit 1 bis 30 Kohlenstoffatomen oder eine aromatische Gruppe,
X ein Anion, das aus der Gruppe der Halogenide,
Phosphate, Acetate, Lactate, C₂-C₆-Alkylsulfate,
Alkylsulfonate oder Alkylarylsulfonate ausgewählt ist;
- quaternären Ammoniumsalzen des Imidazolins;
- quaternären Diammoniumsalzen der Formel (III): 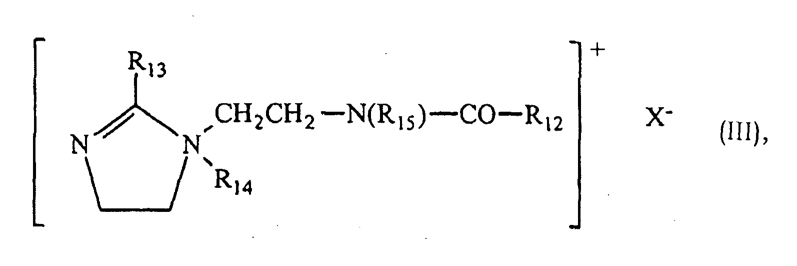 in der bedeuten:
R₁₆ eine aliphatische Gruppe mit etwa 16 bis 30 Kohlenstoffatomen;
R₁₇, R₁₈, R₁₉, R₂₀ und R₂₁, die gleich oder verschieden sind, ein Wasserstoffatom oder eine Alkylgruppe mit 1 bis 4 Kohlenstoffatomen und
X ein Anion, das aus der Gruppe der Halogenide, Acetate, Phosphate, Nitrate und Methylsulfate ausgewählt ist;
- quaternären Ammoniumsalzen, die mindestens eine Esterfunktion enthalten.

12. Zusammensetzung zur kosmetischen Behandlung der Haare nach Anspruch 10 oder 11, **dadurch gekennzeichnet, dass** das kationische Tensid unter Behenyltrimethylammoniumchlorid und Cetyltrimethylammoniumchlorid ausgewählt ist.

13. Zusammensetzung zur kosmetischen Behandlung der Haare nach einem der vorhergehenden Ansprüche, **dadurch gekennzeichnet, dass** sie das kationische Tensid oder die kationischen Tenside in einem Mengenanteil von 0, 1 bis 20 Gew.-%, bevorzugt von 0,2 bis 10 Gew.-% und noch bevorzugter von 0,5 bis 8 Gew.-% enthält, bezogen auf das Gesamtgewicht der Zusammensetzung.

14. Zusammensetzung zur kosmetischen Behandlung der Haare nach einem der vorhergehenden Ansprüche, **dadurch gekennzeichnet, dass** das Polyolefin mit einem oder mehreren polaren Teilen einen apolaren Polyolefinteil besitzt, der mindestens 40 Kohlenstoffatome und vorzugsweise 60 bis 700 Kohlenstoffatome aufweist.

15. Zusammensetzung zur kosmetischen Behandlung der Haare nach Anspruch 14, **dadurch gekennzeichnet, dass** der apolare Polyolefinteil ausgewählt ist unter Oligomeren, Polymeren und/oder Copolymeren von Ethylen, Propylen, 1-Buten, Isobuten, 1-Penten, 2-Methyl-1-buten, 3-Methyl-1-buten, 1-hexen, 1-Hepten, 1-Octen, 1-Decen, 1-Undecen, 1-Dodecen, 1-Tridecen, 1-Tetradecen, 1-Pentadecen, 1-Hexadecen, 1-Heptadecen und 1-Octadecen.

16. Zusammensetzung zur kosmetischen Behandlung der Haare nach einem der vorhergehenden Ansprüche, **dadurch gekennzeichnet, dass** der polare Teil anionisch, kationisch, nicht-ionisch, zwitterionisch oder amphoter ist.

17. Zusammensetzung zur kosmetischen Behandlung der Haare nach einem der vorhergehenden Ansprüche, **dadurch gekennzeichnet, dass** der polare Teil besteht aus Polyalkylenglycolen, Polyalkyleniminen, Carbonsäuren oder Dicarbonsäuren, ihren Anhydriden oder ihren Derivaten wie Estern, Amiden und Salzen, sowie Gemischen dieser Verbindungen.

18. Zusammensetzung zur kosmetischen Behandlung der Haare nach Anspruch 17, **dadurch gekennzeichnet, dass** der polare Teil ausgewählt ist aus der Gruppe, die umfasst:
Polyoxyethylen, Bernsteinsäure oder Bernsteinsäureanhydrid,
Ester oder Amide von Bernsteinsäure oder
Bernsteinsäureanhydrid, Alkalimetallsalze oder
Erdalkalimetallsalze oder organische Salze von Bernsteinsäure oder Bernsteinsäureanhydrid oder partielle Salze von Monoestern oder Monoamiden von Bernsteinsäure oder Bernsteinsäureanhydrid.

19. Zusammensetzung zur kosmetischen Behandlung der Haare nach einem der vorhergehenden Ansprüche, **dadurch gekennzeichnet, dass** das Polyolefin mit einem oder mehreren polaren Teilen ein Polyisobutylen mit gegebenenfalls modifizierter Bernsteinsäure-Endgruppe ist.

20. Zusammensetzung zur kosmetischen Behandlung der Haare nach Anspruch 17, **dadurch gekennzeichnet, dass** das Polyolefin mit einem oder mehreren polaren Teilen das Reaktionsprodukt von Maleinsäureanhydrid mit Polyisobutylen ist.

21. Zusammensetzung zur kosmetischen Behandlung der Haare nach einem der vorhergehenden Ansprüche, **dadurch gekennzeichnet, dass** sie das Polyolefin oder die Polyolefine mit einem oder mehreren polaren Teilen in einem Mengenanteil von 0,01 bis 10 Gew.-% und bevorzugt von 0,1 bis 5 Gew.-% enthält, bezogen auf das Gesamtgewicht der Zusammensetzung.

22. Zusammensetzung zur kosmetischen Behandlung der Haare nach einem der vorhergehenden Ansprüche, **dadurch gekennzeichnet, dass** das C₁₂₋₃₀-Alkylmonoglycosid oder das C₁₂₋₃₀-Alkylpolyglycosid unter entsprechenden Verbindungen ausgewählt ist, deren Alkylgruppe 16 bis 24 Kohlenstoffatome aufweist.

23. Zusammensetzung zur kosmetischen Behandlung der Haare nach einem der vorhergehenden Ansprüche, **dadurch gekennzeichnet, dass** sie das C₁₂₋₃₀-Alkylmonoglycosid oder die C₁₂₋₃₀-Alkylmonoglycoside oder das C₁₂-₃₀-Alkylpolyglycosid oder die C₁₂₋₃₀-Alkylpolyglycoside in einem Mengenanteil von 0,01 bis 10 Gew.-% und bevorzugt von 0,02 bis 5 Gew.-% enthält, bezogen auf das Gesamtgewicht der Zusammensetzung.

24. Zusammensetzung zur kosmetischen Behandlung der Haare nach einem der vorhergehenden Ansprüche, **dadurch gekennzeichnet, dass** das Gewichtsverhältnis Öl(e)/Polyolefin(e) mit einem oder mehreren polaren Teilen im Bereich von 3 bis 100 und bevorzugt im Bereich von 10 bis 75 liegt.

25. Zusammensetzung zur kosmetischen Behandlung der Haare nach einem der vorhergehenden Ansprüche, **dadurch gekennzeichnet, dass** sie ferner mindestens einen C₁₄₋₃₀-Fettalkohol enthält.

26. Zusammensetzung zur kosmetischen Behandlung der Haare nach Anspruch 25, **dadurch gekennzeichnet, dass** der C₁₄₋₃₀-Fettalkohol unter Myristylalkohol, Cetylalkohol, Stearylalkohol, Arachidylalkohol, Behenylalkohol und Erucylalkohol ausgewählt ist.

27. Zusammensetzung zur kosmetischen Behandlung der Haare nach Anspruch 25 oder 26, **dadurch gekennzeichnet, dass** sie den Fettalkohol oder die Fettalkohole in einem Mengenanteil von weniger als 10 Gew.-% enthält, bezogen auf das Gesamtgewicht der Zusammensetzung.

28. Zusammensetzung zur kosmetischen Behandlung der Haare nach einem der vorhergehenden Ansprüche, **dadurch gekennzeichnet, dass** das kosmetisch akzeptable Medium Wasser oder ein Gemisch von Wasser mit mindestens einem kosmetisch akzeptablen Lösungsmittel umfasst.

29. Zusammensetzung zur kosmetischen Behandlung der Haare nach Anspruch 28, **dadurch gekennzeichnet, dass** das kosmetisch akzeptable Lösungsmittel unter niederen C₁-C₄-Alkoholen, Polyolen, Polyolethern, C₅-C₁₀-Alkanen, C₃₋₄-Ketonen, C₁-C₄-Alkylacetaten, Dimethoxyethan und Diethoxyethan ausgewählt ist.

30. Zusammensetzung zur kosmetischen Behandlung der Haare nach einem der vorhergehenden Ansprüche, **dadurch gekennzeichnet, dass** sie ferner mindestens ein Siliconöl in einem Mengenanteil von weniger als 10 Ges.-% enthält, bezogen auf das Gesamtgewicht der Zusammensetzung.

31. Zusammensetzung zur kosmetischen Behandlung der Haare nach einem der vorhergehenden Ansprüche, **dadurch gekennzeichnet, dass** sie mindestens ein Additiv enthält, das ausgewählt ist unter kationischen, anionischen, nichtionischen oder amphoteren Polymeren; natürlichen oder synthetischen anionischen, amphoteren, zwitterionischen, nichtionischen oder kationischen polymeren Verdickungsmitteln, die gegebenenfalls Assoziativpolymere darstellen; nichtpolymeren Verdickungsmitteln; Perlglanzmitteln; Trübungsmitteln; Sonnenfiltern; Parfums; Färbemitteln; organischen oder anorganischen Partikeln; Konservierungsmitteln sowie Mitteln zur Stabilisierung des pH-Werts.

32. Verfahren zur kosmetischen Behandlung der Haare, **dadurch gekennzeichnet, dass** eine Zusammensetzung zur kosmetischen Behandlung nach einem der vorhergehenden Ansprüche auf die Haare aufgebracht wird.

33. Verwendung einer Zusammensetzung zur kosmetischen Behandlung nach einem der Ansprüche 1 bis 31 zur Konditionierung der Haare.

34. Verwendung einer Zusammensetzung zur kosmetischen Behandlung nach Anspruch 33 als Après-Shampoo.
